# EUROPEAN PATENT SPECIFICATION

(11) **EP 4 171 801 B1**
(45) Date of publication and mention of the grant of the patent: **10.06.2026**
(21) Application number: 21736592.3
(22) Date of filing: 25.06.2021
(51) Int. Cl.: B01J 21/04, B01J 23/50, B01J 23/58, B01J 23/68, B01J 35/61, B01J 35/63, B01J 35/64, B01J 35/66, B01J 37/00, B01J 37/08, C07D 301/10, C01F 7/44, C04B 35/111, C04B 38/06, C04B 111/00

(54) **TABLETED ALPHA-ALUMINA CATALYST SUPPORT**
TABLETTIERTER ALPHA-ALUMINIUMOXID-KATALYSATORTRÄGER
SUPPORT DE CATALYSEUR EN ALPHA-ALUMINE EN FORME DE TABLETTE

(30) Priority: 26.06.2020 EP 20182584; 26.06.2020 EP 20182577; 26.06.2020 EP 20182569
(43) Date of publication of application: 03.05.2023
(73) Proprietor: BASF SE, 67056 Ludwigshafen am Rhein (DE)
(72) Inventor: CHOI, Sung Yeun, 67056 Ludwigshafen (DE); KARPOV, Andrey, 67056 Ludwigshafen (DE); WALSDORFF, Christian, 67056 Ludwigshafen (DE); DUYCKAERTS, Nicolas, 67056 Ludwigshafen (DE); AMAKAWA, Kazuhiko, 67056 Ludwigshafen (DE); HUBACH, Patrick, 67056 Ludwigshafen (DE); KHARAS, Karl C., Iselin, NJ 08830 (US)
(74) Representative: BASF IP Association
(86) International application number: PCT/EP2021/067503
(87) International publication number: WO 2021/260185

(56) References cited:
- EP-A1- 3 639 924
- WO-A1-2006/122948
- CN-A- 109 499 558

## Description

The present invention relates to a tableted catalyst support, a process for producing a tableted alpha-alumina catalyst support, a compacted body obtained by tableting a free-flowing feed mixture, a shaped catalyst body for producing ethylene oxide by gas-phase oxidation of ethylene, and a process for producing ethylene oxide by gas-phase oxidation of ethylene.

Alumina (Al₂O₃) is ubiquitous in supports and/or catalysts for many heterogeneous catalytic processes. Some of these catalytic processes occur under conditions of high temperature, high pressure and/or high water-vapor pressure. For example, in the industrial gas-phase oxidation of ethylene to ethylene oxide, heterogeneous catalysts comprising silver deposited on a porous alumina support are typically used.

It is well known that alumina has a number of crystalline phases such as alpha-alumina (often denoted as α-alumina or α-Al₂O₃), gamma-alumina (often denoted as γ-alumina or γ-Al₂O₃) as well as a number of alumina polymorphs. alpha-Alumina is the most stable at high temperatures, but has the lowest surface area.

gamma-Alumina has a very high surface area. This is generally believed to be because the alumina molecules are in a crystalline structure that is not very densely packed. gamma-Alumina constitutes a part of the series known as activated aluminas or transition aluminas, so-called because it is one of a series of aluminas that can undergo transition to different polymorphs. Unfortunately, when gamma-alumina is heated to high temperatures, the structure of the atoms collapses such that the surface area decreases substantially. The densest crystalline form of alumina is alpha-alumina.

alpha-Alumina supports are typically obtained by extrusion of a precursor material comprising alpha-alumina and subsequent heat treatment of the extrudate. For example, the exemplified carriers of WO 2006/133183 A2 were obtained from extrudates of alpha-alumina precursor materials optionally comprising small amounts of boehmite, which extrudates were dried and heated at temperatures above 1400 °C.

In an extrusion process, a powdery precursor material is mixed with a liquid and further components such as pore-formers, peptizing agents and auxiliary agents to form a paste or dough using, e.g., a kneader or a mixer. In such a process, it can be difficult to control the agglomerate or particle size of the ingredient materials. This is due to the forces employed in mixing, but also due to the extrusion step itself. Severe challenges may be encountered when attempting to scale-up such an extrusion process from lab-scale to production-scale equipment.

Generally, the paste or dough is also prone to aging. This means that properties can change over the duration of an industrial production process, making the extrusion process and physical properties of the obtained product difficult to control. Other problems arise when mineral acids such as nitric acid or hydrochloric acid are used as peptizing agents. In particular, such mineral acids may cause corrosion issues or require additional measures to be taken in the subsequent heat treatment of the shaped alumina bodies.

During extrusion, the paste or dough is pressed through a die via a piston press or an extruder to obtain a shaped body defined in two dimensions, i.e., by its cross-section. The third dimension, i.e., the length of the shaped body, may be controlled by cutting the shaped body perpendicular to the direction of extrusion or in an angled fashion. The extrudate is suitably cut into the desired length while still wet. A relatively wide distribution of lengths between shaped bodies produced by extrusion is often observed. Various cutting devices are known and used in the industry. However, in order to control length distribution, both the cutting frequency and extrusion velocity needs tight control and alignment. When an increased number of shaped bodies with more complex geometries are extruded, such as hollow cylinders or shapes with more than one opening bore, it is also difficult to avoid shape deformation of the shaped body faces where the cutting takes place. The shaped bodies easily distort at the location of the cut, or openings get deformed or smeared and partially closed.

Moreover, due to the malleable nature of the extrusion paste, the extrusion process induces aberrations from an ideal symmetry, by bending or curling of the extrudates. In the case of shaped bodies with bores extending from one surface to the opposite surface of the shaped body, these aberrations lead to a reduction in effective cross-section.

Such aberrations are undesirable, as they induce increased pressure loss in a gas-phase reactor as typically used for the oxidation of ethylene to ethylene oxide. Moreover, the operation of an ethylene oxide plant is generally based on calculations, e.g., computational fluid dynamics (CFD) calculations. Such calculations are less reliable when the catalyst bodies exhibit significant aberrations in length and geometry.

WO 99/48606 A describes supports obtained by tableting a mixture of pseudo-boehmite and gamma-alumina and subsequent calcination at 500 to 800 °C.

WO2006/122948 A1 describes shaped alpha-alumina bodies for the use as inert material in exothermic reactions. These shaped bodies are obtained by heat treatment of compacted bodies obtained from a mixture of gamma-alumina and pseudoboehmite. The porous characteristics of these shaped bodies are not discussed in detail. The reference does not suggest the use of the shaped alpha-alumina bodies as catalyst carrier.

EP-A- 3 639 924 discloses shaped alpha-alumina catalyst supports for catalysts used in ethylene oxide production. The document states that the supports may be made by tableting.

Direct tableting of alpha-alumina may be more difficult, probably due to the high hardness, high brittleness, poor plasticity, and comparatively low surface area of alpha-alumina and the absence of functional groups, acidic and basic in nature, which are available in transition aluminas or alumina hydrates.

To carry out the heterogeneously catalyzed gas-phase oxidation of ethylene to ethylene oxide, a mixture of ethylene and an oxygen-comprising gas, such as air or pure oxygen, is generally passed through a plurality of tubes which are arranged in a reactor in which a packing of shaped catalyst bodies is present. Catalyst performance is typically characterized by selectivity, activity, longevity of catalyst selectivity and activity, and mechanical stability. Selectivity is the molar fraction of the converted olefin yielding the desired olefin oxide. Even small improvements in selectivity and the maintenance of selectivity over longer time yield huge dividends in terms of process efficiency.

For the internal surfaces of a porous supported catalyst to be utilized effectively, the feed gases must diffuse through the pores to reach the internal surfaces, and the reaction products must diffuse away from those surfaces and out of the catalyst body. In a process for producing ethylene oxide by gas-phase oxidation of ethylene, diffusion of ethylene oxide molecules out of the catalyst bodies may be accompanied by undesired consecutive reactions induced by the catalyst, such as isomerization to acetaldehyde followed by complete combustion to carbon dioxide, which reduces the overall selectivity of the process. Average molecular pore residence times and thus the extent to which undesired consecutive reactions occur are influenced by the catalyst's pore structure.

Hence, the catalytic performance is influenced by the catalyst's pore structure, which is essentially determined by the pore structure of the catalyst support. The term "pore structure" is understood to relate to the arrangement of void spaces within the support matrix, including sizes, size distribution, shapes and interconnectivity of pores. It can be characterized by various methods such as mercury porosimetry, nitrogen sorption or computer tomography. H. Giesche, "Mercury Porosimetry: A General (Practical) Overview, Part. Part. Syst. Charact. 23 (2006), 9-19, provides helpful insights with regard to mercury porosimetry.

It is an object of this invention to provide an alpha-alumina catalyst support with high geometrical precision. High geometrical precision allows for a more homogeneous reactor loading and pressure drop across reactor tubes used in commercial multi-tubular reactors, e.g., in ethylene oxide production. The alpha-alumina catalyst support should also display a high overall pore volume, thus allowing for impregnation with a high amount of silver, while exhibiting a surface area sufficiently large so as to provide optimal dispersion of catalytically active species, in particular metal species.

The pore structure is determined by factors including size, size distribution and shape of the grains composing the matrix of the support. It has now been found that highly porous transition aluminas having a low bulk density are useful starting materials for the production of alpha-alumina catalyst supports with beneficial pore structure, in particular transition aluminas having relatively high pore volume and large pore diameters. Such transition aluminas are suitable for shaping via the tableting process to obtain geometrically accurate supports with high total pore volume.

It is understood that, where applicable, all embodiments described for one of the aspects of the invention, i.e., the tableted catalyst support, the process for preparing the tableted alpha-alumina catalyst support, the compacted body obtained by tableting a free-flowing feed mixture, the shaped catalyst body for producing ethylene oxide by gas-phase oxidation of ethylene, or the process for producing ethylene oxide by gas-phase oxidation of ethylene, also apply to all other aspects.

The tableted catalyst support typically has a total pore volume of at least 0.40 mL/g, as determined by mercury porosimetry, preferably at least 0.45 mL/g, more preferably at least 0.50 mL/g, most preferably at least 0.55 mL/g. The tableted catalyst support preferably has a total pore volume of in the range of 0.40 to 1.2 mL/g, more preferably in the range of 0.45 to 1.0 mL/g, most preferably in the range of 0.50 to 0.80 mL/g. Mercury porosimetry may be performed using a Micrometrics AutoPore V 9600 mercury porosimeter (140 degrees contact angle, 485 dynes/cm Hg surface tension, 61,000 psia max head pressure). The mercury porosity is determined according to DIN 66133 herein, unless stated otherwise.

Preferably, a significant proportion of the total pore volume of the tableted catalyst support is contained in pores with a diameter in the range of 0.1 to 1 µm. Without wishing to be bound by theory, it is believed that pores with a diameter in the range of 0.1 to 1 µm provide a particularly suitable environment for catalytic conversion after application of a catalytic species, e.g., via impregnation. The pores are small enough to provide a large surface area, while being large enough for allowing quick diffusion of starting materials and obtained products, thus allowing for high activity and selectivity of catalysts based on such a catalyst support. Pores with a larger diameter are believed to not contribute significantly to the total surface area, thus providing less efficient reaction spaces. Pores with a diameter smaller than 0.1 µm are believed to hinder diffusion of the obtained products, which prolongs exposure of the products to the catalytic species and induces consecutive reactions, thus lowering the selectivity.

The tableted catalyst support typically has a pore volume contained in pores with a diameter in the range of 0.1 to 1 µm of at least 25% of the total pore volume, as determined by mercury porosimetry. Preferably, the tableted catalyst support has a pore volume contained in pores with a diameter in the range of 0.1 to 1 µm of at least 30% of the total pore volume, more preferably at least 40% of the total pore volume, most preferably at least 45% of the total pore volume, such as at least 50% of the total pore volume.

Preferably, the pore volume contained in pores with a diameter of less than 0.1 µm constitutes less than 5% of the total pore volume of the catalyst support, as determined by mercury porosimetry, more preferably less than 1%, most preferably less than 0.1%.

The tableted catalyst support preferably has a BET surface in the range of 0.5 to 5.0 m²/g. Preferably, the tableted catalyst support has a BET surface area in the range of 0.5 to 4.5 m²/g, more preferably 1.0 to 4.5 m²/g, most preferably 1.0 to 4.0 m²/g. Herein, the BET surface area is determined in accordance with DIN ISO 9277 using nitrogen physisorption conducted at 77 K.

The tableted catalyst support usually comprises at least 85 wt.-% of alpha-alumina, based on the total weight of the support, preferably at least 90 wt.-%, more preferably at least 95 wt.-%, most preferably at least 97.5 wt.-%. The amount of the alpha-alumina can for example be determined via X-ray diffraction analysis.

In one embodiment, the tableted catalyst support comprises at least 85 wt.-% of alpha-alumina, based on the total weight of the support, the support having
- a total pore volume of at least 0.40, mL/g, as determined by mercury porosimetry;
- a BET surface area in the range of 0.5 to 5.0 m²/g;
- a pore volume contained in pores with a diameter of less than 0.1 µm of less than 5% of the total pore volume, as determined by mercury porosimetry; and
- a pore volume contained in pores with a diameter in the range of 0.1 to 1 µm of at least 25% of the total pore volume, as determined by mercury porosimetry.

The shape of the tableted catalyst support is not particularly limited, as long as it is accessible by a conventionally known tableting press of the punch-and-die type. The shapes of the tableted catalyst support are generally such that each is composed of a circumferential surface, which corresponds to the internal wall of the die cavity, and a top face side surface and a bottom face side surface, which correspond to the operative heads of the punches. It is also possible that the upper punch and lower punch come together during the tableting process. In this case, no discrete circumferential and face side surfaces are formed. Thus, tableted catalyst supports having an outer shape of, e.g., a sphere or an ellipsoid may be obtained.

In a preferred embodiment, the tableted catalyst support has a first face side surface, a second face side surface and a circumferential surface, the circumferential surface extending essentially in parallel to a longitudinal axis of the catalyst support. The longitudinal axis of the catalyst support is understood to be an axis extending from the first face side surface to the second face side surface. Typically, the tableted catalyst support is Cₙ-symmetric, such as C₂- to C₇-symmetric or has full rotational symmetry, with respect to the longitudinal axis.

The circumferential surface extending essentially in parallel to the longitudinal axis of the catalyst support is understood to include slight deviations from the ideal geometry, such as a slightly conical shape of the circumferential surface. The circumferential surface extending "essentially in parallel" to the longitudinal axis of the catalyst support is understood to mean that the circumferential surface extends parallel to the longitudinal axis with less than 5° of deviance, preferably less than 2.5° of deviance, more preferably less than 1° of deviance.

In another embodiment, the geometry of the tableted catalyst support may be modified such that the circumferential surface no longer extends in parallel to the longitudinal axis and be structured with cylindrical and/or curved or conical segments of various or varying angles. For example, the geometry may be modified such that the geometric shape of its outer surface no longer corresponds to that of a circular cylinder but rather at least partly to that of a frustocone or a frustosphere. The related die has an upper circular cylinder wherein the upper punch is slidable, a lower circular cylinder having a cross-sectional area lower than that of the circular cylinder, wherein the lower punch is slidable, and an intermediate section which widens from the bottom upward. Owing to the altered geometric conditions in the removal of the shaped precursor body formed from the die bore by lifting the lower punch, the friction between the inner wall of the die bore and the outer surface of the shaped precursor body can essentially be eliminated.

In one embodiment, at least one passageway extends from the first face side surface to the second face side surface of the tableted catalyst support. When the shaped body comprises multiple passageways, the longitudinal axes of the passageways are typically parallel. The circumferential surfaces of the passageways are preferably "essentially parallel" to the longitudinal axes of the passageways. This is understood to include embodiments wherein the passageways are at least partially conical, rather than cylindrical. Such a slightly conical shape may be desirable to allow for better ejection of a compacted body in a tableting process. The circumferential surfaces of the passageways preferably extend parallel to the longitudinal axis with less than 5° of deviance, preferably less than 2.5° of deviance, more preferably less than 1° of deviance.

The tableted catalyst support may be flat-topped or have domed ends, i.e., at least one of the first face side surface and the second face side surface is curved. The dome ratio to the straight part of the catalyst support (i.e., dome lengths divided by the height of straight part) may in the range 0.10 to 0.40. Curved face side surfaces such as domed face side surfaces reduce the sharpness of corners of the support, allowing for less abrasion and hence less catalyst dust.

In one embodiment, the catalyst support is in the shape of hollow cylinders or annular tablets wherein at least one face side surface is rounded to the outer edge, preferably both face side surfaces. In one embodiment, the catalyst support may be in the shape of hollow cylinders or annular tablets having a central passageway extending from the first face side surface to the second face side surface of the tableted catalyst support, wherein at least one face side surface is rounded both to the outer edge and to the edge of the central passageway, so that the catalyst support does not comprise right-angled edges. Such shapes have been described, e.g., in US 6,518,220 B2.

In another embodiment the catalyst support may be in a shape such as described in US 9,409,160 B2 wherein the shaped catalyst body has the form of a cylinder with a base, a cylinder surface, a cylinder axis and at least one continuous opening (a passageway that extends from the first face side surface to the second face side surface of the tableted catalyst support) running parallel to the cylinder axis, and the base of the cylinder has at least four lobes.

The catalyst support may also be in a shape such as described in WO 2012/091898 A2, having at least three lobes, a first end, a second end, a wall between the ends and a non-uniform radius of transition at the intersection an end and the wall.

In a preferred embodiment the catalyst support has more than one passageway extending from the first face side surface to the second face side surface of the tableted catalyst support. Such shapes are known in the art, as described in the following.

For example, US 5,861,353 A describes catalysts and catalyst carriers in the form of cylindrical granules, characterized in that each granule displays at least three through-bores (passageways that extend from the first face side surface to the second face side surface of the tableted catalyst support) having axes which are substantially parallel to each other and to the axes of the granule, and substantially equidistant from each other.

US 9,138,729 B2 describes a shaped catalyst that has an essentially cylindrical body having a longitudinal axis, wherein the cylindrical body has at least two parallel internal holes (passageways that extend from the first face side surface to the second face side surface of the tableted catalyst support) which are essentially parallel to the cylinder axis of the body and go right through the body, and wherein the internal holes have a round or oval cross section.

WO 2020/108872 A1 describes a shaped catalyst body for producing ethylene oxide by gas-phase oxidation of ethylene, comprising silver deposited on a porous refractory support, the shaped catalyst body having a first face side surface, a second face side surface and a circumferential surface, a cylinder structure with n void spaces running in the cylinder periphery along the cylinder height to form an n-lobed structure, wherein n is 2, 3, 4, 5 or 6, n passageways extending from the first face side surface to the second face side surface, each passageway being assigned to one lobe, wherein neighboring passageways are arranged essentially equidistantly to each other, an n-fold rotational symmetry, a shortest distance A between two neighboring passageways in the range of 1.0 to 2.0 mm and a shortest distance B between each passageway and the circumferential surface in the range of 1.1 to 2.0 mm.

The tableting process allows the accurate manufacture of catalyst supports, i.e., the manufacture of a plurality of catalyst supports having relatively little deviations in outer dimensions. Such supports are geometrically nearly identical, which allows for a better calculability of their behavior in reaction processes and a lower pressure loss in, e.g., gas-phase catalysis.

In one embodiment, the invention provides a plurality of catalyst supports as described above, wherein the supports have a height (length) with no more than a 5% sample standard deviation s from the mean height. Preferably, the supports have a height with no more than a 5% sample standard deviation s from the mean support height, most preferably a height with no more than a 3% sample standard deviation s from the mean support height.

In one embodiment, the invention provides a plurality of catalyst supports as described above, wherein the supports have an outer diameter with no more than a 1% sample standard deviation s from the mean outer diameter. Preferably, the supports have an outer diameter with no more than a 0.7% sample standard deviation s from the mean support height, most preferably a height with no more than a 0.5% sample standard deviation s from the mean support height. The "outer diameter" is understood to mean the diameter of the circumscribed circle of the cross-section perpendicular to the support height, i.e., the diameter of the smallest circle that completely contains the support cross-section within it.

The sample standard deviation s is understood to be the corrected sample standard deviation, i.e., the standard deviation after application of Bessel's correction. The sample standard deviation s of a plurality of n catalyst supports may be calculated as follows. First, the mean (average) height and/or outer diameter of n catalyst supports is determined. The deviations of each value from the mean are calculated, and the result of each deviation is squared. The sum of the squared deviations is divided by the value of (n - 1), and the square root of the resulting value constitutes the sample standard deviation. The obtained result is reported relative to the sample mean, i.e., the obtained value is divided by the sample mean value and is expressed as a percentage of the sample mean. This may also be referred to as the relative sample standard deviation s. To obtain a meaningful result, the height of a significant number of catalyst supports, such as at least 100 catalyst supports, should be determined.

The invention relates to a process for producing a tableted alpha-alumina catalyst support, which comprises
i) forming a free-flowing feed mixture comprising, based on inorganic solids content, at least 50 wt.-% of a transition alumina;
ii) tableting the free-flowing feed mixture to obtain a compacted body; and
iii) heat treating the compacted body at a temperature of at least 1100 °C, preferably at least 1300 °C, more preferably at least 1400 °C, in particular at least 1450 °C, to obtain the tableted alpha-alumina catalyst support.

In a preferred embodiment, the obtained tableted alpha-alumina catalyst support is a tableted catalyst support as described above.

The feed mixture is a free-flowing feed mixture, i.e. a mixture in which the particles do not cling together. The flow properties are determined using the vessel method of Klein in Klein, K.; Seifen, Ole, Fette, Wachse, 94, 849 (1968). This is a method that uses a series of outflow vessels wherein each has a different opening in the bottom. The material to be tested is added to the vessel and the outflow from the opening in the bottom of the vessel is studied. The qualification of the flow properties is determined by the smallest opening through which the powder can still flow. Materials in the classes numbered 1 to 4 are usually considered as free-flowing. Typical examples of free-flowing feed mixtures are powders. The powders may vary in degrees of fineness.

The free-flowing feed mixture comprises, based on inorganic solids content, at least 50 wt.-% of a transition alumina. Preferably, the free-flowing feed mixture comprises, based on inorganic solids content, at least 60 wt.-%, more preferably at least 70 wt.-% of the transition alumina, such as at least 80 wt.-% or at least 90 wt.-%, in particular 95 to 100 wt.-%.

The term "transition alumina" is understood to mean an alumina comprising a metastable alumina phase, such as a gamma-, delta-, eta-, theta-, kappa- or chi-alumina phase. Preferably, the transition alumina comprises at least 80 wt.-%, preferably at least 90 wt.-%, most preferably at least 95 wt.-%, such as 95 to 100 wt.-%, of a phase selected from gamma-alumina, delta-alumina and/or theta-alumina, based on the total weight of the transition alumina, in particular a phase selected from gamma-alumina and/or delta-alumina.

The transition alumina is typically in the form of a powder. Transition aluminas are commercially available and may be obtained via thermal dehydration of hydrated aluminum compounds, in particular aluminum hydroxides and aluminum oxy-hydroxides. Suitable hydrated aluminum compounds include naturally occurring and synthetic compounds, such as aluminum trihydroxides (Al(OH)₃) like gibbsite, bayerite and nordstrandite, or aluminum oxy-monohydroxides (AlOOH) like boehmite, pseudoboehmite and diaspore.

By progressively dehydrating hydrated aluminum compounds, lattice rearrangements are affected. For example, boehmite can be converted to gamma-alumina at about 450 °C, gamma-alumina can be converted to delta-alumina at about 750 °C, and delta-alumina can be converted to theta-alumina at about 1,000 °C. When heating at above 1,000 °C, transition aluminas are converted to alpha-alumina.

It is believed that the morphological properties of the resulting transition aluminas are primarily dependent on the morphological properties of the hydrated aluminum compounds from which they are derived. Accordingly, Busca, "The Surface of Transitional Aluminas: A Critical Review", Catalysis Today, 226 (2014), 2-13, describes that alumina derived from a variety of pseudoboehmites have differing pore volumes and pore size distributions, despite the pseudoboehmites having similar surface areas (160 ~ 200 m²/g).

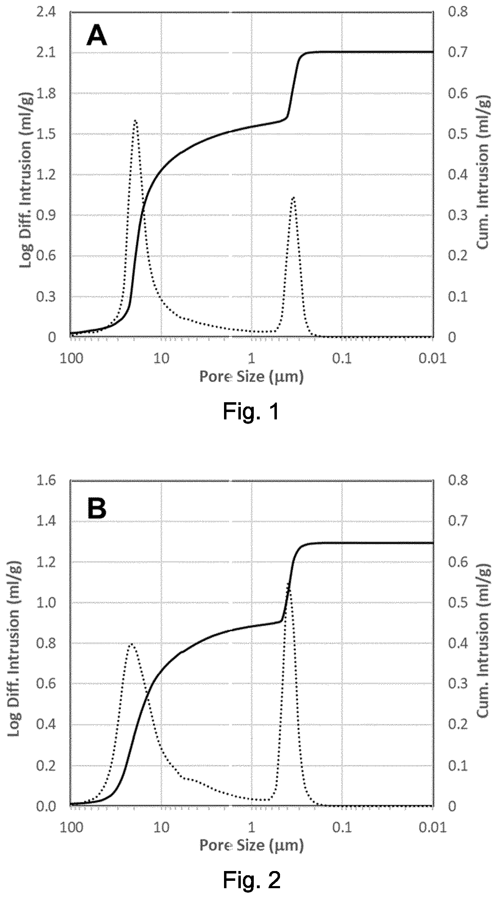

In a preferred embodiment, the transition alumina comprises non-platelet crystals. The term "non-platelet" refers to any form other than platelet form, for example elongated forms such as rods or needles, or forms having approximately the same dimensions in all three spatial directions. In a preferred embodiment, the transition alumina comprises non-platelet shaped crystals, such as rod-shaped crystals as described in, e.g., WO 2010/068332 A1, or block-shaped crystals as described in, e.g., Busca, "The Surface of Transitional Aluminas: A Critical Review", Catalysis Today, 226 (2014), 2-13, see Fig. 2c, 2d and 2e as compared to Fig. 2a, 2b and 2f. Preferably, the average crystal size of the transition alumina is at least 5 nm, preferably at least 7 nm, most preferably at least 10 nm, as determined via the Scherrer equation from XRD patterns.

Various synthetic methods of obtaining crystalline boehmitic alumina with high pore volume and large surface area with high thermal stability are known, e.g., from WO 00/09445 A2, WO 01/02297 A2, WO 2005/014482 A2 and WO 2016/022709 A1. For example, WO 2016/022709 A1 describes boehmitic alumina with an average pore diameter of 115 to 166 Å, a bulk density of 250 to 350 kg/m³ and a pore volume of 0.8 to 1.1 m³/g, prepared by precipitation of basic aluminum salts with acidic alumina salts under controlled pH and temperature. Transition aluminas produced by thermal treatment of these boehmitic aluminas and having the properties defined in the present claims are particularly suitable transition alumina for use in the process of the invention.

Prior to heat treatment, the hydrated aluminum compounds may be washed, e.g., with demineralized water, so as to reduce impurities and allow for obtaining a high purity transition alumina. For example, crystalline boehmite obtained from gibbsite by a hydrothermal process according to Chen et al., J. Solid State Chem., 265 (2018), 237 to 243, is preferably washed prior to heat treatment.

High purity transition aluminas are preferred so as to limit the content of impurities such as sodium or silicon in the catalyst support. High purity transition aluminas may be obtained, e.g., via the so-called Ziegler process, sometimes referred to as ALFOL process, and variants thereof as described in Busca, "The Surface of Transitional Aluminas: A Critical Review", in Catalysis Today, 226 (2014), 2-13. Other processes based on the precipitation of aluminates such as sodium aluminate tend to yield transition aluminas with relatively high amounts of impurities, such as sodium.

Transition aluminas used in the present invention preferably have a total content of alkali metals, e.g., sodium and potassium, of at most 1500 ppm, more preferably at most 600 ppm and most preferably 10 ppm to 200 ppm, relative to the total weight of the transition alumina. Various washing methods are known that allow for the reduction of the alkali metal content of the transition alumina and/or the catalyst support obtained therefrom. Washing can include washing with a base, an acid, water or other liquids.

US 2,411,807 A describes that the sodium oxide content in alumina precipitates may be reduced by washing with a solution containing hydrofluoric acid and another acid. WO 03/086624 A1 describes carrier pretreatment with an aqueous lithium salt solution so as to remove sodium ions from the surface of a carrier. US 3,859,426 A describes the purification of refractory oxides such as alumina and zirconia by repetitive rinsing with hot deionized water. WO 2019/039930 describes a purification method of alumina in which metal impurities were removed by extraction with an alcohol.

Besides alkali metals, the levels of other naturally occurring impurities are preferably controlled as well.

Transition aluminas used in the present invention preferably have a total content of alkaline-earth metals, such as calcium and magnesium, of at most 2,000 ppm, more preferably at most 600 ppm and most preferably at most 400 ppm, relative to the total weight of the transition alumina.

Transition aluminas used in the present invention preferably have a content of silicon of at most 10,000 ppm, preferably at most 2,000 ppm and most preferably at most 700 ppm, relative to the total weight of the transition alumina.

Transition aluminas used in the present invention preferably have a content of iron of at most 1,000 ppm, more preferably at most 600 ppm and most preferably at most 300 ppm, relative to the total weight of the transition alumina.

Transition aluminas used in the present invention preferably have a content of metals different from those mentioned above, such as titanium, zinc, zirconium, and lanthanum, of at most 1,000 ppm, more preferably at most 400 ppm and most preferably at most 100 ppm, relative to the total weight of the transition alumina.

The transition alumina preferably has a loose bulk density of at most 600 g/L. The term "loose bulk density" is understood to be the "freely settled" or "poured" density. The "loose bulk density" thus differs from the "tapped density", where a defined mechanical tapping sequence is applied and a higher density is typically obtained. The loose bulk density may be determined by pouring the transition alumina into a graduated cylinder, suitably via a funnel, taking care not to move or vibrate the graduated cylinder. The volume and weight of the alumina are determined. The loose bulk density is determined by dividing the weight in grams by the volume in liters.

A low loose bulk density may be indicative of a high porosity and a high surface area. Preferably, the transition alumina has a loose bulk density in the range of 50 to 600 g/L, more preferably in the range of 100 to 550 g/L, most preferably 150 to 500 g/L, in particular 200 to 500 g/L or 200 to 450 g/L.

The transition alumina has a pore volume of at least 0.6 mL/g. Preferably, the transition alumina has a pore volume of 0.6 to 2.0 mL/g or 0.65 to 2.0 mL/g, more preferably 0.7 to 1.8 mL/g, most preferably 0.8 to 1.6 mL/g.

The transition alumina has a median pore diameter of at least 15 nm. The term "median pore diameter" is used herein to indicate the median pore diameter by surface area, i.e., the median pore diameter (area) is the pore diameter at the 50^{th} percentile of the cumulative surface area graph. Preferably, the transition alumina has a median pore diameter of 15 to 500 nm, more preferably 20 to 450 nm, most preferably 20 to 300 nm, such as 20 to 200 nm.

Mercury porosimetry and nitrogen sorption are widely used to characterize the pore structure for porous materials, because these methods enable the determination of porosity and pore size distribution in one step. The two techniques are based on different physical interactions and optimally cover specific ranges of pore size.

In many cases, nitrogen sorption constitutes a sufficiently accurate determination method, particularly for smaller pores. Hence, the pore volume and the median pore diameter of transition aluminas may be determined by nitrogen sorption. Nonetheless, larger pores may be underrepresented by nitrogen sorption.

Nitrogen sorption measurements may be performed using a Micrometrics ASAP 2420. Nitrogen porosity is determined according to DIN 66134 herein, unless stated otherwise. Barrett-Joyner-Halenda (BJH) pore size and volume analysis is carried out to obtain the total pore volume ("BJH desorption cumulative pore volume") and median pore diameter ("BJH desorption average pore diameter").

Mercury porosimetry may be performed using a Micrometrics AutoPore V 9600 mercury porosimeter (140 degrees contact angle, 485 dynes/cm Hg surface tension, 61,000 psia max head pressure). For total pore volume and median pore diameter of transition aluminas, data is taken in the pore diameter range of 3 nm to 1 µm.

For adequate accuracy, the reported pore volume and the median pore diameter of transition aluminas are from nitrogen sorption if the median pore diameter from mercury porosimetry is less than 50 nm; or the reported pore volume and the median pore diameter of transition aluminas are from mercury porosimetry if the median pore diameter from mercury porosimetry is 50 nm or more.

In order to avoid falsification of the results, nitrogen sorption measurements and mercury porosimetry should be carried out on samples treated so as to remove physically adsorbed species, such as moisture, from the samples. A suitable method is described below.

The transition alumina typically has a BET surface area in the range of 20 to 500 m²/g. The BET method is a standard, well-known method and widely used method in surface science for the measurements of surface areas of solids by physical adsorption of gas molecules. The BET surface is determined according to DIN ISO 9277 using nitrogen physisorption conducted at 77 K herein, unless stated otherwise. The terms "BET surface area" and "surface area" are used equivalently herein, unless noted otherwise.

The BET surface area of the transition alumina may vary over a relatively large range and may be adjusted by varying the conditions of the thermal dehydration of the hydrated aluminum compounds by which the transition alumina may be obtained. Preferably, the transition alumina has a BET surface area in the range of 20 to 200 m²/g, more preferably 50 to 200 m²/g or 50 to 150 m²/g.

Suitable transition aluminas are commercially available. In some instances, such commercially available transition aluminas are classified as "medium porosity aluminas" or, in particular, "high porosity aluminas". Suitable transition aluminas include products of the Puralox^{®} TH and Puralox^{®} TM series, both from Sasol, and products of the Versal VGL series from UOP.

The transition alumina may be used in its commercially available ("unmilled") form. This commercial form of alumina comprises agglomerates (secondary particles) of the individual particles or grains (primary particles). For example, a commercial alumina particle with a mean (secondary) particle diameter (e.g., D₅₀) of 25 µm may comprise sub-micron sized primary particles. The mean particle diameter (D₅₀) as referred to herein is understood to mean the particle diameter (D₅₀) of secondary alumina particles.

Unmilled transition alumina powder typically has a D₅₀ particle diameter of 10 to 100 µm, preferably 20 to 50 µm. In addition, transition alumina may be used which has been subjected to grinding to break down the particles to a desired size. Suitably, the transition alumina may be milled in the presence of a liquid, and is preferably milled in the form of a suspension. Alternatively, grinding may be affected by dry ball-milling. Milled transition alumina powder typically has a D₅₀ particle diameter of 0.5 to 8 µm, preferably 1 to 5 µm. The particle size of transition alumina may be measured by laser diffraction particle size analyzers, such as a Malvern Mastersizer 2000 using water as a dispersing medium. The method includes dispersing the particles by ultrasonic treatment, thus breaking up secondary particles into primary particles. This sonication treatment is continued until no further change in the D₅₀ value is observed, e.g., after sonication for 3 min.

In a preferred embodiment, the transition alumina comprises at least 50 wt.-%, preferably 60 to 90 wt.-% of a transition alumina having an average particle size of 10 to 100 µm, preferably 20 to 50 µm, based on the total weight of transition alumina. Optionally, the transition alumina may comprise a transition alumina having an average particle size of 0.5 to 8 µm, preferably 1 to 5 µm, such as at most 50 wt.-%, preferably 10 to 40 wt.-%, based on the total weight of transition alumina.

The free-flowing feed mixture comprises, based on inorganic solids content, at most 40 wt.-% of an alumina hydrate. Preferably, the free-flowing feed mixture comprises, based on inorganic solids content, 1 to 40 wt.-% of the alumina hydrate, more preferably 1 to 30 wt.-%, most preferably 1 to 20 wt.-%, such as 3 to 18 wt.-%.

The term "alumina hydrate" is understood to relate to hydrated aluminum compounds as described above, in particular aluminum hydroxides and aluminum oxy-hydroxides. A discussion of the nomenclature of aluminas may be found in K. Wefers and C. Misra, "Oxides and Hydroxides of Aluminum", Alcoa Laboratories, 1987. Suitable hydrated aluminum compounds include naturally occurring and synthetic compounds, such as aluminum trihydroxides (Al(OH₃) like gibbsite, bayerite and nordstrandite, or aluminum oxy-monohydroxides (AlOOH) like boehmite, pseudoboehmite and diaspore.

Preferably, the alumina hydrate comprises gibbsite, bayerite, boehmite, and/or pseudoboehmite, in particular boehmite and/or pseudoboehmite. In a preferred embodiment, the total amount of boehmite and pseudoboehmite constitutes at least 80 wt.-%, more preferably at least 90 wt.-% and most preferably at least 95 wt.-%, such as 95 to 100 wt.-%, of the alumina hydrate. In an especially preferred embodiment, the amount of boehmite constitutes at least 80 wt.-%, more preferably at least 90 wt.-% and most preferably at least 95 wt.-%, such as 95 to 100 wt.-%, of the alumina hydrate.

Suitable alumina hydrates are commercially available and include products of the Pural^{®} series from Sasol, preferably products of the Pural^{®} TH and Pural^{®} TM series, and products of the Versal^{®} series from UOP.

Without wishing to be bound by theory, it is believed that the presence of alumina hydrate increases the mechanical stability of the support. In particular, it is believed that nano-sized, highly dispersible alumina hydrates suitable for colloidal applications, such as boehmites of the Disperal^{®} or Dispal^{®} series from Sasol exhibit high binding forces and can enhance the mechanical stability of the support especially efficiently. In general, using such nano-sized, highly dispersible alumina hydrates to improve mechanical stability may allow for relatively lower BET-surface areas at given heat treatment conditions.

Alumina hydrate may be partially or fully replaced by suitable alternative aluminum compounds while essentially retaining the mechanical stability of the support. Such suitable alternative aluminum compounds include aluminum alkoxides like aluminum ethoxide and aluminum isopropoxide, aluminum nitrate, aluminum acetate and aluminum acetylacetonate.

The free-flowing feed mixture optionally comprises a liquid. The presence, type and amount of the liquid may be chosen in accordance with the desired handling properties of the free-flowing feed mixture. Incorporation of a liquid may be beneficial in order to avoid segregation phenomena in the free-flowing feed mixture. In order not to affect the free-flowing nature of the feed mixture, it is preferred that the free-flowing feed mixture has a limited liquid content of, e.g., less than 15 wt.-%, preferably less than 10 wt.-%, more preferably less than 5 wt.-%, in particular less than 1 wt.-%, based on the solids content of the free-flowing material. The suitable amounts depends on the porosity and water uptake of the solid components in the powder. In a preferred embodiment, the free-flowing feed mixture is free of liquid components or essentially free of liquid components, i.e., an amount of less than 0.1 wt.-%, in particular less than 0.05 wt.-%, based on the solids content of the free-flowing material. In another embodiment, higher amounts of a liquid such as water can be added, however this may negatively impact the flowability of the powder.

The liquid is typically selected from water, in particular de-ionized water, and/or an aqueous solution comprising soluble and/or dispersible compounds selected from salts, such as ammonium acetate and ammonium carbonate; acids, such as formic acid, nitric acid, acetic acid and citric acid; bases, such as ammonia, triethylamine and methylamine; surfactants such as triethanolamine, poloxamers, fatty acid esters, and alkyl polyglucosides; submicron-sized particles, including metal oxides such as silica, titania and zirconia; clays; and/or polymer particles such as polystyrene and polyacrylates. The liquid is preferably water, most preferably de-ionized water.

The liquid will mostly be adsorbed liquid (or moisture) rather than free inter-grain liquid. The amount of liquid comprised in the free-flowing feed mixture can be determined as the weight loss after heating at 130 °C for one hour.

The free-flowing feed mixture may comprise further components, which may be processing aids or which are purposively introduced to adjust the physical properties of the final catalyst support. Further components include pore-forming materials, lubricants, organic binders, and/or inorganic binders.

The free-flowing feed mixture may comprise organic materials such as pore-forming materials, lubricants, and organic binders in a total amount of 1.0 to 60 wt.-%, preferably 3 to 50 wt.-%, based on the total weight of the free-flowing feed mixture.

The free-flowing mixture may comprise lubricants and organic binders in amounts 1.0 to 10 wt.-%, preferably 3 to 8 wt.-%, based on the total weight of the free-flowing mixture. Advantageously, the free-flowing mixture used in the inventive process requires relatively small amounts of lubricant.

Pore-forming materials may be used to provide additional and/or wider pores in the support. The additional pore volume of wider pores can also advantageously allow for a more efficient impregnation of the support during the production of a catalyst. Preferably, pore-forming materials are essentially completely removed during heat treatment of the compacted bodies. The pore-forming function may be achieved by different mechanisms, such as combustion (i.e., burning) in the presence of oxygen, decomposition, sublimation, or volatilization.

Suitable pore-forming materials include
- thermally decomposable materials such as oxalic acid, malonic acid, ammonium carbonate or ammonium bicarbonate;
- burnout materials, e.g., thermally combustible biomaterials such as acacia, sawdust, and flours, in particular ground nut shell flours, such as flours of pecan shells, cashew shells, walnut shells or filbert shells; and/or
- organic polymers such as
   - polysaccharides, such as starch, gums, cellulose and cellulose derivatives, including substituted celluloses such as methyl cellulose, ethyl cellulose, and carboxy ethyl cellulose, and cellulose ethers;
   - polyolefins, like polyethylene and polypropylene;
   - aromatic hydrocarbon polymers, like polystyrene;
   - polycarbonates, such as poly(propylene carbonate); and
   - lignins;
- carbonaceous materials such as
   - graphite;
   - powdered carbonaceous compounds such as coke, or activated carbon powders; and
   - milled or unmilled carbon fibers.

In one embodiment, the pore-forming material is selected from water-soluble pore-forming materials, i.e., pore-forming materials having an aqueous solubility of at least 80 g/L at 20 °C. Suitable water-soluble pore-forming materials include oxalic acid and/or malonic acid. Such water-soluble pore-forming materials are suitably applied in the particulate state, i.e., an undissolved state.

Thermally decomposable materials such as oxalic acid, malonic acid, ammonium bicarbonate or ammonium carbonate decompose upon thermal treatment and break down into volatile smaller molecules, which may or may not be combustible. For example, malonic acid decomposes upon thermal treatment to predominantly yield acetic acid and carbon dioxide. Such thermally decomposable materials may offer certain advantages in industrial processes, as these materials generally can be obtained from industrial sources with a degree of purity that they do not introduce contaminants into the support.

In order to avoid formation of a potentially explosive atmosphere, heat treatment of the compacted body is preferably conducted under an atmosphere of reduced oxygen content, such as at most 10 vol.-% or at most 5 vol.-% of oxygen, when thermally decomposable materials are used. If the thermal decomposition occurs at relatively low temperatures, the process may be safely controlled well below the ignition temperature of potentially combustible molecules formed upon decomposition of the decomposable materials. This may allow safe operation of thermal treatment even at relatively high concentrations of oxygen in the atmosphere inside the apparatus for thermal treatment. In this case, an atmosphere of air may be used.

In one embodiment, the pore-forming material has a median diameter (D₅₀) of less than 600 µm, preferably less than 500 µm, more preferably less than 300 µm. In another embodiment, the pore-forming material has a median diameter (D₅₀) of at least 1 µm, preferably at least 5 µm, more preferably at least 10 µm. In another embodiment the particle size distribution of a commercial pore forming material can be controlled by milling or crushing and sieving or screening steps. Preferably, the pore-forming material has a narrow pore size distribution width. One of the common values to characterize the distribution width is the span value, defined as (D₉₀-D₁₀)/D₅₀. Preferably, the span value is less than 10, more preferably less than 5 and most preferably less than 3.

Lubricants lower the adhesive friction between the compacted body and the inner wall of the tableting die.

Suitable lubricants include
- graphite;
- petroleum jelly, mineral oil, or grease;
- fatty acids, such as stearic acid or palmitic acid; salts of fatty acids, such as stearates like potassium stearate, magnesium stearate and aluminum stearate or palmitates like potassium palmitate, magnesium palmitate and aluminum palmitate; fatty acid derivatives, such as esters of fatty acids, in particular esters of saturated fatty acids, such as stearate esters like methyl and ethyl stearate; and/or
- malleable organic solids such as waxes like paraffin wax, cetyl palmitate.

It is preferable that the lubricant does not introduce inorganic contaminations into the catalyst support. Among the above-mentioned lubricants, graphite, stearic acid, aluminum stearate, and combinations thereof are preferred.

Organic binders, which sometimes are also referred to as "temporary binders" may be used to maintain the integrity of the "green" phase, i.e. the unfired phase, in which the mixture is formed into compacted bodies. Preferably, organic binders are essentially completely removed during heat treatment of the compacted bodies.

Suitable organic binders include
- polyvinyl lactam polymers, such as polyvinylpyrrolidones, or vinylpyrrolidone copolymers such as vinylpyrrolidone-vinyl acetate copolymers;
- alcohols, in particular polyols such as glycol or glycerol; and/or
- polyalkylene glycols, such as polyethylene glycol.

When solid, non-malleable organic binders such as graphite and/or solid, non-malleable lubricants are used, the particle size of these organic binders and lubricants is preferably smaller than that of alumina raw materials, such as the transition alumina and alumina hydrates. Typically, the median diameter (D₅₀) of solid, non-malleable organic binders and solid, non-malleable lubricants is less than 100 µm, preferably less than 50 µm, more preferably less than 30 µm, and most preferably less than 10 µm. Preferably, the span value is less than 7, more preferably less than 5 and most preferably less than 3.

Advantageously, pore formers and processing aids, e.g., organic binders and lubricants exhibit a low ash content. The term "ash content" is understood to relate to the incombustible component remaining after combustion of the organic materials in air at high temperature, i.e. after heat treatment of the compacted bodies. The ash content is preferably below 0.1 wt.-%, relative to the total weight of organic materials.

Moreover, pore formers and processing aids, e.g., organic binders and lubricants preferably do not form significant amounts of volatile further combustible components, such as carbon monoxide, ammonia or combustible organic compounds, upon heat treating the compacted bodies, i.e. upon thermal decomposition or combustion. An appropriate safety concept is preferably applied for the combustion or decomposition process step.

Inorganic binders are permanent binders, which contribute to the adequate bonding of alumina particles and enhance the mechanical stability of the shaped alpha-alumina bodies. Inorganic binders include those which upon calcination yield exclusively aluminum oxide. For the purposes of this application, these inorganic binders are termed intrinsic inorganic binders. Such intrinsic inorganic binders include alumina hydrate as discussed above.

Extrinsic inorganic binders, on the other hand, do not exclusively yield aluminum oxide upon calcination. Suitable extrinsic inorganic binders are understood to be any of the inorganic species conventionally used in the art, e.g., silicon-containing species such as silica or silicates, including clays such as kaolinite, or metal hydroxides, metal carbonates, metal nitrates, metal acetates or metal oxides such as zirconia, titania, or alkali metal oxides. Since extrinsic inorganic binders introduce contaminants which may be detrimental to catalyst performance, they are preferably comprised in controlled amounts. Preferably, the precursor material includes extrinsic inorganic binders in amounts of 0.0 to 5.0 wt.-%, preferably 0.05 to 1.0 wt.-%, based on the inorganic solids content of the precursor material. In a preferred embodiment, the precursor material does not comprise an extrinsic inorganic binder.

The free-flowing feed mixture is typically obtained by dry-mixing its components, and then optionally adding the liquid.

The free-flowing feed mixture is tableted to obtain a compacted body, i.e., the free-flowing feed mixture is shaped into a compacted body via tableting.

Tableting is a process of press agglomeration. The free-flowing feed mixture is introduced into a pressing tool having a die between two punches and compacted by uniaxial compression and shaped to give a solid compacted body. Tableting may be divided into four parts: metered introduction, compaction (elastic deformation), plastic deformation and ejection. Tableting is carried out, for example, on rotary presses or eccentric presses.

The outer surface of the tableted catalyst support is composed of a circumferential surface, which corresponds to the internal wall of the die cavity, and a first face side surface and a second face side surface, which correspond to the operative heads of the punches. The tableted catalyst support may be flat-topped or have domed ends, i.e., at least one of the first face side surface and the second face side surface is curved. Curved face side surfaces may be obtained by using, e.g., a concave lower and/or upper punch. If desired, the upper punch and/or lower punch may comprise projecting pins to form internal passageways. It is also possible to provide the pressing punches with a plurality of movable pins, so that a punch can, for example, be made with four pins to create shaped bodies with four holes (passageways). Typical design features of such tools may be found in, e.g., US 8,865,614 B2.

The pressing tool is chosen in accordance with the desired geometrical dimensions of the compacted body. The size and shape of the compacted body and thus of the catalyst is selected to allow a suitable packing of the catalyst bodies obtained from compacted bodies in a reactor tube. The catalysts obtained from the compacted bodies suitable for the catalysts of the invention are preferably used in reactor tubes with a length from 6 to 14 m and an inner diameter from 20 mm to 50 mm. In general, the support is comprised of individual bodies having a maximum extension in the range of 3 to 20 mm, such as 4 to 15 mm, in particular 5 to 12 mm. The maximum extension is understood to mean the longest straight line between two points on the outer circumference of the support.

The shape of the compacted bodies is not especially limited, and may be in any technically feasible form, depending, e.g., on the forming process. For example, the support may be a solid tablet or a hollow tablet, such as a hollow cylinder. In another embodiment, the support may be characterized by a multilobe structure. A multilobe structure is meant to denote a cylinder structure which has a plurality of void spaces, e.g., grooves or furrows, running in the cylinder periphery along the cylinder height. Generally, the void spaces are arranged essentially equidistantly around the circumference of the cylinder.

The pressing force during tableting affects compaction of the free-flowing feed mixture and thus, e.g., the density and/or mechanical stability of the compacted body. In practice, it has been found to be useful to set the lateral compressive strength of the tableted catalyst support in a targeted manner by selection of the appropriate pressing force and to check this by random sampling. For the purposes of the present invention, the lateral compressive strength is the force which fractures the tableted catalyst support located between two flat parallel plates, with the two flat parallel end faces of the catalyst support being at right angles to the flat parallel plates.

To improve tableting properties, the free-flowing feed mixture may be subjected to further processing, e.g., by sieving, pre-heating and/or pre-granulation, i.e. pre-compaction. For pre-granulation, a roll compactor, such as a Chilsonator^{®} from Fitzpatrick, may be used.

Further information regarding tableting, in particular with regard to pre-granulation, sieving, lubricants and tools, may be found in WO 2010/000720 A2. More information on tableting is provided in the Handbook of Powder Technology, Chapter 16: Tabletting, K. Pitt and C. Sinka, Vol 11, 2007, p. 735 to 778.

The invention further provides a compacted body obtained by tableting a free-flowing feed mixture which comprises, based on inorganic solids content, at least 50 wt.-% of a transition alumina having a loose bulk density of at most 600 g/L, a pore volume of at least 0.6 mL/g, and a median pore diameter of at least 15 nm, as described above.

Notably, tableted compacted bodies, i.e., green bodies, are more mechanically stable than extruded bodies. Tableted compacted bodies thus advantageously allow for better handling and reduced deformation prior to heat treatment.

The compacted body is heat treated to form the tableted alpha-alumina catalyst support. Prior to heat treatment, the compacted body may be dried, in particular when the free-flowing feed mixture comprises a liquid. Suitably, drying is performed at temperatures in the range of 20 to 400 °C, in particular 30 to 300 °C, such as 70 to 150 °C. Drying is typically performed over a period of up to 100 h, preferably 0.5 h to 30 h, more preferably 1 h to 16 h.

Drying may be performed in any atmosphere, such as in an oxygen-containing atmosphere like air, in nitrogen, or in helium, or in mixtures thereof, preferably in air. Drying is usually carried out in an oven. The type of oven is not especially limited. For example, stationary circulating air ovens, revolving cylindrical ovens or conveyor ovens may be used. Heat may be applied directly and / or indirectly.

Preferably, flue gas (vent gas) from a combustion process having a suitable temperature is used in the drying step. The flue gas may be used in diluted or non-diluted form to provide direct heating and to remove evaporated moisture and other components liberated from the shaped bodies. The flue gas is typically passed through an oven as described above. In another preferred embodiment, off-gas from a heat treatment process step is used for direct heating.

Drying and heat treatment may be carried out sequentially in separate apparatuses and may be carried out in a batch-wise or continuous process. Intermittent cooling may be applied. In another embodiment, drying and heat treatment are carried out in the same apparatus. In a batch process, a time-resolved temperature ramp (program) may be applied. In a continuous process, a space-resolved temperature-ramp (program) may be applied, e.g., when the shaped bodies are continuously moved through areas (zones) of different temperatures.

Preferably, measures of heat-integration as known in the art are applied in order to improve energy efficiency. For example, relatively hotter off-gas from one process step or stage can be used to heat the feed gas, apparatus or shaped bodies in another process step or stage by direct (admixing) or indirect (heat-exchanger) means. Likewise, heat integration may also be applied to cool relatively hotter off-gas streams prior to further treatment or discharge.

The shaped bodies are heat treated to obtain the tableted alpha-alumina catalyst support. Thus, the heat treatment temperature and duration are sufficient to convert at least part of the transition alumina to alpha-alumina, meaning that at least part of the metastable alumina phases of the transition alumina is converted to alpha-alumina.

The obtained tableted catalyst support typically comprises a high proportion of alpha-alumina, for example at least 85 wt.-%, preferably at least 90 wt.-%, more preferably at least 95 wt.-%, most preferably at least 97.5 wt.-%, based on the total weight of the support. The amount of the alpha-alumina can for example be determined via X-ray diffraction analysis.

Heat treatment is performed at a temperature of at least 1100 °C, such as at least 1300 °C, more preferably at least 1400 °C, in particular at least 1450 °C. Preferably, heat treatment is performed at an absolute pressure in the range of 0.5 bar to 35 bar, in particular in the range of 0.9 to 1.1 bar, such as at atmospheric pressure (approximately 1013 mbar). Typical total heating times range from 0.5 to 100 h, preferably from 2 to 20 h.

A suitable flow rate for the heated gas may be in the range of, e.g., 1 to 1,000 Nm³/h, 10 to 1,000 Nm³/h, 15 to 500 Nm³/h or 20 to 300 Nm³/h per kg of compacted bodies. In a continuous process, the term "kg of compacted bodies" is understood to mean the amount of compacted bodies (in kg/h) multiplied by the time (in hours) that the gas stream is directed over the compacted bodies. It has been found that when the gas stream is directed over higher amounts of compacted bodies, e.g., 15 to 150 kg of compacted bodies, the flow rate may be chosen in the lower part of the above-described ranges, while achieving the desired effect.

Heat treatment is usually carried out in a furnace. The type of furnace is not especially limited. For example, furnaces such as stationary circulating air furnaces, revolving cylindrical furnaces or conveyor furnaces, or kilns such as rotary kilns or tunnel kilns, in particular roller hearth kilns, may be used. In one embodiment, heat treatment constitutes directing a heated gas stream over the compacted bodies. Heat treatment can be carried out in a pass-through mode or with at least partial recycling of the heated gas.

Determining the temperature of the heated compacted bodies directly may pose practical difficulties. Hence, when a heated gas is directed over the compacted bodies during heat treatment, the temperature of the heated compacted bodies is considered to be the temperature of the gas immediately after the gas has passed over the compacted bodies. In a practical embodiment, the compacted bodies are placed on a suitable surface, such as a wire mesh or perforated calcination belt, and the temperature of the gas is measured by one or more thermocouples positioned adjacent to the opposite side of the compacted bodies which first comes into contact with the gas. The thermocouples are suitably placed close to the compacted bodies, e.g., at a distance of 1 to 30 mm, such as 1 to 3 mm or 15 to 20 mm from the compacted bodies.

Heat treatment may be performed in any atmosphere, such as in an oxygen-containing atmosphere like air, in nitrogen, or in helium, or in mixtures thereof. Preferably, in particular when the compacted bodies contain a thermally decomposable material or a burnout material, heat treatment is at least in part or entirely carried out in an oxidizing atmosphere, such as in an oxygen-containing atmosphere like air.

As described above, pore formers and processing aids, e.g., organic binders and lubricants, preferably do not form significant amounts of volatile further combustible components, such as carbon monoxide or combustible organic compounds, upon heat treatment of the shaped bodies. An explosive atmosphere may further be avoided by limiting the oxygen concentration in the atmosphere during heat treatment, e.g., to an oxygen concentration below the limiting oxygen concentration (LOC) with respect to the further combustible components. The LOC, also known as minimum oxygen concentration (MOC), is the limiting concentration of oxygen below which combustion is not possible.

Suitably, lean air or a gaseous recycle stream with limited oxygen content may be used along with a stream for oxygen make-up, which also compensates for gaseous purge streams. In an alternative approach, an explosive atmosphere can be avoided by limiting the rate of formation of further combustible components. The rate of formation of further combustible components may be limited by heating to the heat treatment temperature via a slow temperature ramp, or by heating in a step-wise manner. When heating in a step-wise manner, the temperature is suitably held for several hours at the approximate combustion temperature, then heating to temperatures of 1000 °C. In a continuous heat treatment process, the feed rate of the shaped bodies to the heat treatment device, e.g., the furnace, may also be controlled so as to limit the rate of formation of further combustible components.

In another embodiment, depending on the nature of organic materials present in the shaped bodies, such as pore-forming materials, lubricants, and organic binders, the temperature may be controlled below the ignition temperature of the organic material or their decomposition products until all relevant organic material has been safely removed, so as to mitigate risks of an explosion. This may be applicable when a thermally decomposable material, e.g., malonic acid is present.

Depending on the nature of pore-forming materials, lubricants, and organic binders, a waste-gas treatment may be applied in order to purify any off-gas obtained during heat treatment. Preferably, an acidic or alkaline scrubber, a flare or catalytic combustion, a DeNOx treatment or combinations thereof may be used for off-gas treatment.

Preferably, heating takes place in a step-wise manner. In step-wise heating, the shaped bodies may be placed on a high purity and inert refractory saggar which is moved through a furnace with multiple heating zones, e.g., 2 to 8 or 2 to 5 heating zones. The inert refractory saggar may be made of alpha-alumina or corundum, in particular alpha-alumina.

The tableted catalyst support may comprise impurities, such as sodium, potassium, magnesium, calcium, silicon, iron, titanium and/or zirconium. Such impurities may be introduced by components of the free-flowing feed mixture, in particular inorganic binders or mechanical stability enhancers. In one embodiment, the tableted catalyst support comprises
- a total amount of up to 1,500 ppmw of sodium and potassium;
- up to 2,000 ppmw of calcium;
- up to 1,000 ppmw of magnesium;
- up to 10,000 ppmw of silicon;
- up to 1,000 ppmw of titanium;
- up to 1,000 ppmw of iron; and/or
- up to 10,000 ppmw of zirconium;
relative to the total weight of the support.

A low content of sodium is preferred in order to prevent segregation of the supported metal and to prevent alteration of the supported component.

The invention further relates to a shaped catalyst body for producing ethylene oxide by selective gas-phase oxidation (epoxidation) of ethylene, i.e. an epoxidation catalyst, comprising at least 15 wt.-% of silver, relative to the total weight of the shaped catalyst body, deposited on a tableted alpha-alumina catalyst support described above or on a tableted alpha-alumina catalyst support obtained in the process described above.

The shaped catalyst body typically comprises 15 to 70 wt.-% of silver, preferably 20 to 60 wt.-% of silver, more preferably 25 to 50 wt.-% or 30 to 50 wt.-% of silver, relative to the total weight of the shaped catalyst body. A silver content in this range allows for a favorable balance between turnover induced by each shaped catalyst body and cost-efficiency of preparing the shaped catalyst body.

Besides silver, the shaped catalyst body may comprise one or more promoting species. A promoting species denotes a component that provides an improvement in one or more of the catalytic properties of the catalyst when compared to a catalyst not containing said component. The promoting species can be any of those species known in the art that function to improve the catalytic properties of the silver catalyst. Examples of catalytic properties include operability (resistance to runaway), selectivity, activity, turnover and catalyst longevity.

The shaped catalyst body may comprise a promoting amount of a transition metal or a mixture of two or more transition metals. Suitable transition metals can include, for example, the elements from Groups IIIB (scandium group), IVB (titanium group), VB (vanadium group), VIB (chromium group), VIIB (manganese group), VIIIB (iron, cobalt, nickel groups), IB (copper group), and IIB (zinc group) of the Periodic Table of the Elements, as well as combinations thereof. More typically, the transition metal is an early transition metal, i.e., from Groups IIIB, IVB, VB or VIB, such as, for example, hafnium, yttrium, molybdenum, tungsten, rhenium, chromium, titanium, zirconium, vanadium, tantalum, niobium, or a combination thereof. In one embodiment, the transition metal promoter(s) is (are) present in a total amount from 150 ppm to 5,000 ppm, typically 225 ppm to 4,000 ppm, most typically from 300 ppm to 3,000 ppm, expressed in terms of metal(s) relative to the total weight of the shaped catalyst body.

Of the transition metal promoters listed, rhenium (Re) is a particularly efficacious promoter for ethylene epoxidation high selectivity catalysts. The rhenium component in the shaped catalyst body can be in any suitable form, but is more typically one or more rhenium-containing compounds (e.g., a rhenium oxide) or complexes.

In some embodiments, the shaped catalyst body may include a promoting amount of an alkali metal or a mixture of two or more alkali metals. Suitable alkali metal promoters include, for example, lithium, sodium, potassium, rubidium, cesium or combinations thereof. The amount of alkali metal, e.g. potassium, will typically range from 50 ppm to 5,000 ppm, more typically from 300 ppm to 2,500 ppm, most typically from 500 ppm to 1,500 ppm expressed in terms of the alkali metal relative to the total weight of the shaped catalyst body. The amount of alkali metal is determined by the amount of alkali metal contributed by the tableted catalyst support and the amount of alkali metal contributed by the impregnation solution described below.

Combinations of heavy alkali metals like cesium (Cs) or rubidium (Rb) with light alkali metals like lithium (Li), sodium (Na) and potassium (K) are particularly preferred.

The shaped catalyst body may also include a Group IIA alkaline earth metal or a mixture of two or more Group IIA alkaline earth metals. Suitable alkaline earth metal promoters include, for example, beryllium, magnesium, calcium, strontium, and barium or combinations thereof. The amounts of alkaline earth metal promoters can be used in amounts similar to those used for the alkali or transition metal promoters.

The shaped catalyst body may also include a promoting amount of a main group element or a mixture of two or more main group elements. Suitable main group elements include any of the elements in Groups IIIA (boron group) to VIIA (halogen group) of the Periodic Table of the Elements. For example, the shaped catalyst body can include a promoting amount of sulfur, phosphorus, boron, halogen (e.g., fluorine), gallium, or a combination thereof.

The shaped catalyst body may also include a promoting amount of a rare earth metal or a mixture of two or more rare earth metals. The rare earth metals include any of the elements having an atomic number of 57 to 103. Some examples of these elements include lanthanum (La), cerium (Ce), and samarium (Sm). The amount of rare earth metal promoters can be used in amounts similar to those used for the transition metal promoters.

The shaped catalyst body as described above may be obtained by process comprising
a) impregnating a catalyst support as described above with a silver impregnation solution, preferably under reduced pressure; and optionally subjecting the impregnated catalyst support to drying; and
b) subjecting the impregnated catalyst support to a post-impregnation heat treatment;
wherein steps a) and b) are optionally repeated.

In order to obtain a shaped catalyst body having high silver contents, steps i) and ii) can be repeated several times. In that case it is understood that the intermediate product obtained after the first (or subsequent up to the last but one) impregnation / post-impregnation heat treatment cycle comprises a part of the total amount of target Ag and/or promoter concentrations. The intermediate product is then again impregnated with the silver impregnation solution and post-impregnation heat treated to yield the target Ag and/or promoter concentrations.

Any silver impregnation solution suitable for impregnating a refractory support known in the art can be used. Silver impregnation solutions typically contain a silver carboxylate, such as silver oxalate, or a combination of a silver carboxylate and oxalic acid, in the presence of an aminic complexing agent like a C₁-C₁₀-alkylenediamine, in particular ethylenediamine. Suitable impregnation solutions are described in EP 0 716 884 A2, EP 1 115 486 A1, EP 1 613 428 A1, US 4,731,350 A, WO 2004/094055 A2, WO 2009/029419 A1, WO 2015/095508 A1, US 4,356,312 A, US 5,187,140 A, US 4,908,343 A, US 5,504,053 A and WO 2014/105770 A1. For a discussion of suitable silver impregnation solutions, see also Kunz, C. et al., On the Nature of Crystals Precipitating from Aqueous Silver Ethylenediamine Oxalate Complex Solutions., Z. Anorg. Allg. Chem., 2021, 647, DOI: 10.1002/zaac.202100079.

During post-impregnation heat treatment, liquid components of the silver impregnation solution evaporate, causing a silver compound comprising silver ions to precipitate from the solution and be deposited onto the support. At least part of the deposited silver ions is subsequently converted to metallic silver upon further post-impregnation heating. Preferably, at least 70 mol-% of the silver compounds, preferably at least 90 mol-%, more preferably at least 95 mol-% and most preferably at least 99.5 mol-% or at least 99.9 mol-%, i.e. essentially all of the silver ions, based on the total molar amount of silver in the impregnated catalyst support, respectively. The amount of the silver ions converted to metallic silver can for example be determined via X-ray diffraction (XRD) patterns.

The post-impregnation heat treatment may also be referred to as a calcination process. Any calcination processes known in the art for this purpose can be used. Suitable examples of calcination processes are described in US 5,504,052 A, US 5,646,087 A, US 7,553,795 A, US 8,378,129 A, US 8,546,297 A, US 2014/0187417 A1, EP 1 893 331 A1 or WO 2012/140614 A1. Post-impregnation heat treatment can be carried out in a pass-through mode or with at least partial recycling of the calcination gas.

Post-impregnation heat treatment is usually carried out in a furnace. The type of furnace is not especially limited. For example, stationary circulating air furnaces, revolving cylindrical furnaces or conveyor furnaces may be used. In one embodiment, post-impregnation heat treatment constitutes directing a heated gas stream over the impregnated bodies. The duration of the post-impregnation heat treatment is generally in the range of 5 min to 20 h, preferably 5 min to 30 min.

The temperature of the post-impregnation heat treatment is generally in the range of 200 to 800 °C, preferably 210 to 650 °C, more preferably 220 to 500 °C, most preferably 220 to 350 °C. Preferably, the post-impregnation heating rate in the temperature range of 40 to 200 °C is at least 20 K/min, more preferably at least 25 K/min, such as at least 30 K/min. A high post-impregnation heating rate may be achieved by directing a heated gas over the impregnated refractory support or the impregnated intermediate catalyst at a high gas flow.

A suitable flow rate for the gas may be in the range of, e.g., 1 to 1,000 Nm³/h, 10 to 1,000 Nm³/h, 15 to 500 Nm³/h or 20 to 300 Nm³/h per kg of impregnated bodies. In a continuous process, the term "kg of impregnated bodies" is understood to mean the amount of impregnated bodies (in kg/h) multiplied by the time (in hours) that the gas stream is directed over the impregnated bodies. It has been found that when the gas stream is directed over higher amounts of impregnated bodies, e.g., 15 to 150 kg of impregnated bodies, the flow rate may be chosen in the lower part of the above-described ranges, while achieving the desired effect.

Determining the temperature of the heated impregnated bodies directly may pose practical difficulties. Hence, when a heated gas is directed over the impregnated bodies during post-impregnation heat treatment, the temperature of the heated impregnated bodies is considered to be the temperature of the gas immediately after the gas has passed over the impregnated bodies. In a practical embodiment, the impregnated bodies are placed on a suitable surface, such as a wire mesh or perforated calcination belt, and the temperature of the gas is measured by one or more thermocouples positioned adjacent to the opposite side of the impregnated bodies which first comes into contact with the gas. The thermocouples are suitably placed close to the impregnated bodies, e.g., at a distance of 1 to 30 mm, such as 1 to 3 mm or 15 to 20 mm from the impregnated bodies.

The use of a plurality of thermocouples can improve the accuracy of the temperature measurement. Where several thermocouples are used, these may be evenly spaced across the area on which the impregnated bodies rest on the wire mesh, or the breadth of the perforated calcination belt. The average value is considered to be the temperature of the gas immediately after the gas has passed over the impregnated bodies. To heat the impregnated bodies to the temperatures as described above, the gas typically has a temperature of 220 to 800 °C, more preferably 230 to 550 °C, most preferably 240 to 350 °C.

Preferably, post-impregnation heating takes place in a step-wise manner. In step-wise post-impregnation heating, the impregnated bodies are placed on a moving belt that moves through a furnace with multiple heating zones, e.g., 2 to 8 or 2 to 5 heating zones. Post-impregnation heat treatment is preferably performed in an inert atmosphere, such as nitrogen, helium, or mixtures thereof, in particular in nitrogen.

The invention further relates to a process for producing ethylene oxide by gas-phase oxidation of ethylene, comprising reacting ethylene and oxygen in the presence of a shaped catalyst body as described above.

The epoxidation can be carried out by all processes known to those skilled in the art. It is possible to use all reactors which can be used in the ethylene oxide production processes of the prior art. The epoxidation is preferably carried out in at least one tube reactor, preferably in a shell-and-tube reactor. On a commercial scale, ethylene epoxidation is preferably carried out in a multi-tube reactor that contains several thousand tubes. The catalyst is filled into the tubes, which are placed in a shell that is filled with a coolant.

To prepare ethylene oxide from ethylene and oxygen, it is possible to carry out the reaction under conventional reaction conditions. Inert gases such as nitrogen or gases which are inert under the reaction conditions, e.g. steam, methane, and also optionally reaction moderators, for example halogenated hydrocarbons such as ethyl chloride, vinyl chloride or 1,2-dichloroethane can additionally be mixed into the reaction gas comprising ethylene and molecular oxygen.

The concentration of carbon dioxide in the feed (i.e. the gas mixture fed to the reactor) typically depends on the catalyst selectivity and the efficiency of the carbon dioxide removal equipment. Carbon dioxide concentration in the feed is preferably at most 3 vol.-%, more preferably less than 2 vol.-%, most preferably less than 1 vol.-%, relative to the total volume of the feed.

The reaction or oxidation of ethylene to ethylene oxide is usually carried out at elevated catalyst temperatures. Preference is given to catalyst temperatures in the range of 150 to 350 °C, more preferably 180 to 300 °C, particularly preferably 190 to 280 °C and especially preferably 200 to 280 °C. The present invention therefore also provides a process as described above in which the oxidation is carried out at a catalyst temperature in the range 180 to 300 °C, preferably 200 to 280 °C.

The reaction according to the invention (oxidation) is preferably carried out at pressures in the range of 5 to 30 bar. All pressures herein are absolute pressures, unless noted otherwise. The oxidation is more preferably carried out at a pressure in the range of 5 to 25 bar, such as 10 bar to 24 bar and in particular 14 bar to 23 bar.

It has been found that the physical characteristics of the shaped catalyst body, especially the BET surface area and the pore size distribution have a significant positive impact on the catalyst selectivity. This effect is especially distinguished when the catalyst is operated at very high work rates, i.e., high levels of olefin oxide production.

The process according to the invention is preferably carried out under conditions conducive to obtain a reaction mixture containing at least 2.3 vol.-% of ethylene oxide. In other words, the ethylene oxide outlet concentration (ethylene oxide concentration at the reactor outlet) is preferably at least 2.3 vol.-%. The ethylene oxide outlet concentration is more preferably in the range of 2.5 to 4.0 vol.-%, most preferably in the range of 2.7 to 3.5 vol.-%.

The oxidation is preferably carried out in a continuous process. If the reaction is carried out continuously, the GHSV (gas hourly space velocity) is, depending on the type of reactor chosen, for example on the size/cross-sectional area of the reactor, the shape and size of the catalyst, preferably in the range from 800 to 10,000/h, preferably in the range from 2,000 to 8,000/h, more preferably in the range from 2,500 to 6,000/h, most preferably in the range from 4,500 to 5,500/h, where the values indicated are based on the volume of the catalyst.

The preparation of ethylene oxide from ethylene and oxygen can advantageously be carried out in a recycle process. After each pass, the newly formed ethylene oxide and the by-products formed in the reaction are removed from the product gas stream. The remaining gas stream is supplemented with the required amounts of ethylene, oxygen and reaction moderators and reintroduced into the reactor.

The invention is described in more detail by the accompanying drawings and the subsequent examples.
Fig. 1 shows the cumulative intrusion [mL/g] relative to the pore size diameter [mL/g] of an inventive tableted catalyst support A.
Fig. 2 shows the cumulative intrusion [mL/g] relative to the pore size diameter [mL/g] of an inventive tableted catalyst support B.
Fig. 3 shows the cumulative intrusion [mL/g] relative to the pore size diameter [mL/g] of an inventive tableted catalyst support C.
Fig. 4 shows the cumulative intrusion [mL/g] relative to the pore size diameter [mL/g] of an inventive tableted catalyst support D.
Fig. 5 shows the cumulative intrusion [mL/g] relative to the pore size diameter [mL/g] of an inventive tableted catalyst support E.
Fig. 6 shows the cumulative intrusion [mL/g] relative to the pore size diameter [mL/g] of an inventive extruded catalyst support F.

### Method 1: Nitrogen Sorption

Nitrogen sorption measurements were performed using a Micrometrics ASAP 2420. Nitrogen porosity was determined in accordance with DIN 66134. The sample was degassed at 200 °C for 16 h under vacuum prior to the measurement.

### Method 2: Mercury Porosimetry

Mercury porosimetry was performed using a Micrometrics AutoPore V 9600 mercury porosimeter (140 degrees contact angle, 485 dynes/cm Hg surface tension, 61,000 psia max head pressure). Mercury porosity was determined in accordance with DIN 66133.

Samples were dried at 110 °C for 2 h and degassed under vacuum prior to analysis to remove any physically adsorbed species, such as moisture, from the sample surface.

### Method 3: Loose Bulk Density

The loose bulk density was determined by pouring the transition alumina or alumina hydrate into a graduated cylinder via a funnel, taking care not to move or vibrate the graduated cylinder. The volume and weight of the transition alumina or alumina hydrate were determined. The loose bulk density was determined by dividing the volume in milliliters by the weight in grams.

### Method 4: BET Surface Area

The BET surface area was determined in accordance with DIN ISO 9277 using nitrogen physisorption conducted at 77 K. The surface area was obtained from a 5-point-BET plot. The sample was degassed at 200 °C for 16 h under vacuum prior to the measurement. In the case of shaped alpha-alumina supports, more than 4 g of the sample were applied due to its relatively low BET surface area.

### Method 5: Dimension of Shaped Bodies and Sample Standard Deviation s

The dimensions of the shaped bodies were measured using a digital caliper (Holex 412811). The "length" was the height of the shaped body, i.e., the along the longitudinal axis. The "outer diameter" was the diameter of the circumscribed circle of the cross-section perpendicular to the support height. Geometric precision is described as the sample standard deviation s of length and outer diameter of a plurality of 100 catalyst supports which were calculated as follows. First, the mean (average) length and outer diameter of 100 catalyst supports were determined. The deviations of each length and outer diameter value from the mean were calculated, and the result of each deviations were squared. The sum of the squared deviations is divided by the value of 99 and the square root of the resulting value constitutes the sample standard deviation s of length and outer diameter. The obtained result is reported relative to the sample mean, i.e., the obtained value is divided by the sample mean value and is expressed as a percentage of the sample mean.

### Preparation of Tableted Catalyst Supports A, B, C, D and E

The properties of the alumina raw materials used to obtain porous alpha-alumina catalyst supports are shown in Table 1. The transition aluminas and alumina hydrates were obtained from Sasol (Puralox^{®}, and Pural^{®}) and UOP (Versal^{®}).

**Table 1**

| Transition Aluminas * | | | |
|---|---|---|---|
| | Loose Bulk Density [g/L] | Pore Volume [mL/g] ** | Median Pore Diameter [nm] ** |
| Puralox TM 100/150UF | 150 | 0.88 | 18.4 |
| Puralox TH 200/70 | 300 | 1.23 | 37.4 |
| Versal VGL-15 | 310 | 0.86 | 21.7 |

| Alumina Hydrates * | | | |
|---|---|---|---|
| | Loose Bulk Density [g/L] | Pore Volume [mL/g] ** | Median Pore Diameter [nm] ** |
| Pural TH 200 | 340 | 1.20 | 37.6 |
| Versal V-250 | 360 | 0.79 | 9.9 |

| | | | |
|---|---|---|---|
| * Puralox products are transition aluminas derived from Pural products, i.e. boehmite; Versal VGL-15 is a gamma-alumina derived from Versal V-250, i.e. pseudoboehmite ** determined by nitrogen sorption | | | |

Alumina raw materials, as specified in Table 1, and pore former were mixed with Cutina^{®} HR (hydrogenated castor oil waxy mass from BASF) and Timrex^{®} T44 (graphite from TimCal Graphite & Carbon) as processing aids to obtain a powder mixture. The amounts of all components are shown in Table 2.

The pore formers used are listed in Table 2. Olive stone granule (olive stone granules, BioPowder), pulp granule (Technocel^{®} 200G, CFF) and microcrystalline cellulose bead (MCC 200, Zhongbao Chemicals) were used as received without any pretreatment. The particle size of the pore formers was in the range between 100 µm and 300 µm. Malonic acid (M1296, purity 99.0%, Sigma-Aldrich) was gently ground in a mortar and sieved prior to use. The particles of malonic acid used for the sample preparation were collected in between 60 mesh and 200 mesh.

The powder mixture was subjected to a tableting machine equipped with a hollow cylinder punch having an outer diameter of 6.6 mm and an inner diameter of 3.7 mm. The obtained tablets were thermally treated in a muffle furnace. The furnace temperature was ramped up to 600 °C at a heating rate of 5 °C /min, held at 600 °C for 2 h, then ramped up to 1,464 °C at a heating rate of 2 °C /min and held at 1,464 °C for 4 h. Subsequently, the active heating was switched off and the furnace cooled down to room temperature (about 23 °C) overnight. Heat treatment was performed under lean air with 5 vol.-% of oxygen.

**Table 2**

| Support | Transition Alumina | Alumina Hydrate | Pore Former | Processing Aid |
|---|---|---|---|---|
| A | Puralox TH 200/70 52 g | Pural TH 200 20 g | Olive Stone Granule 75 g | Cutina HR 5 g |
| | Puralox TM 100/150 UF 28 g | | | Timrex T44 3 g |
| B | Puralox TH 200/70 80 g | Pural TH 200 20 g | Pulp Granule 75 g | Cutina HR 5 g |
| | | | | Timrex T44 3 g |
| C | Puralox TH 200/70 80 g | Pural TH 200 20 g | Microcrystalline Cellulose Beads 75 g | Cutina HR 5 g |
| | | | | Timrex T44 3 g |
| D | Versal VGL-15 80 g | Versal V-250 20 | Olive Stone Granule 50 g | Cutina HR 5 g |
| | | | | Timrex T44 3 g |
| E | Puralox TH 200/70 80 g | Pural TH 200 20 g | Malonic Acid 50 g | Cutina HR 5 g |
| | | | | Timrex T44 3 g |

### Preparation of Comparative Extrudate F

A commercial alpha-alumina support for ethylene oxide catalyst was obtained from EXACER s.r.l. (Via Puglia 2 /4, 41049 Sassuolo (MO), Italy), under the lot number COM 46/20. The support was produced by extrusion.

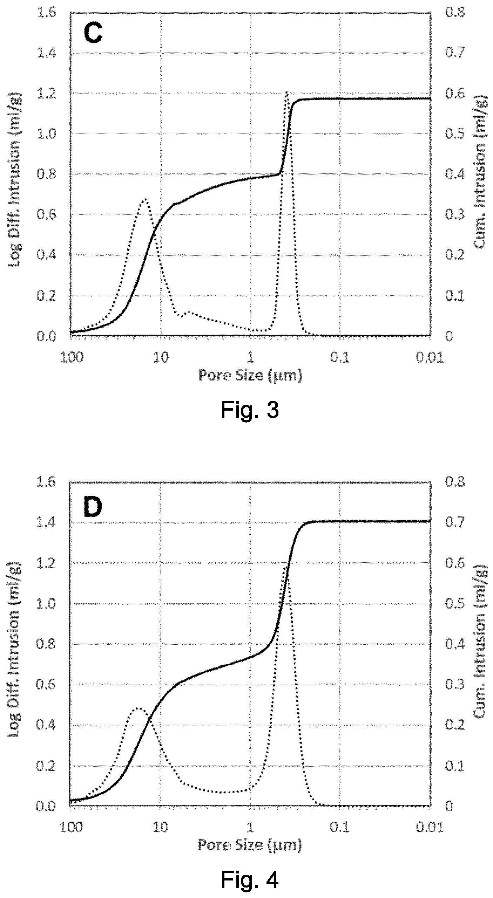

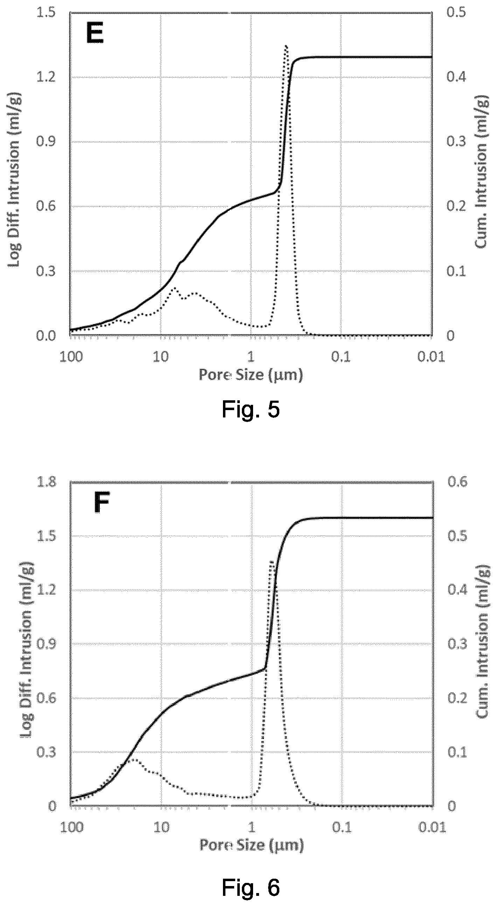

Figs. 1 to 6 show the log differential intrusion and cumulative intrusion relative to the pore size diameter of the supports A to F.

Table 3 shows the physical properties of the supports A to F.

**Table 3.**

| Support | BET Surface Area [m²/g] | Total Pore Volume [mL/g] | Pore Volume Contained in Pores [mL/g] ** (Proportion of the Total Pore Volume) | | | | Sample Standard Deviation s *** | |
|---|---|---|---|---|---|---|---|---|
| | | | < 0.1 µm | 0.1 - 1 µm | 1 - 10 µm | > 10 µm | Length [%] | Outer Diameter [%] |
| A | 2.01 | 0.70 | 0 (0%) | 0.18 (25.7%) | 0.11 (15.7%) | 0.41 (58.6%) | 1.9 | 0.2 |
| B | 2.05 | 0.65 | 0 (0%) | 0.21 (32.3%) | 0.11 (16.9%) | 0.33 (50.8%) | 1.8 | 0.2 |
| C | 2.40 | 0.59 | 0 (0%) | 0.20 (33.9%) | 0.11 (18.6%) | 0.28 (47.5%) | 1.5 | 0.3 |
| D | 2.55 | 0.70 | 0 (0%) | 0.33 (47.2%) | 0.11 (15.7%) | 0.26 (37.1%) | 3.2 | 0.2 |
| E | 1.95 | 0.43 | 0 (0%) | 0.22 (51.2%) | 0.14 (32.5%) | 0.07 (16.3%) | 1.0 | 0.2 |
| F* | 2.01 | 0.53 | 0 (0%) | 0.29 (54.7%) | 0.07 (13.2%) | 0.17 (32.1%) | 5.5 | 2.4 |

| | | | | | | | | |
|---|---|---|---|---|---|---|---|---|
| * comparative example ** determined by mercury porosimetry *** obtained from 100 samples of each support | | | | | | | | |

It is evident that the inventive supports A to E prepared by tableting exhibit a similar BET surface area and total pore volume as comparative support F. Moreover, supports A to E were obtained with significantly higher geometrical precision than comparative support F.

## Claims

1. A process for producing a tableted alpha-alumina catalyst support, which comprises
i) forming a free-flowing feed mixture comprising, based on inorganic solids content, at least 50 wt.-% of a transition alumina, wherein the free-flowing feed mixture further comprises a pore-forming material;
ii) tableting the free-flowing feed mixture to obtain a compacted body; and
iii) heat treating the compacted body at a temperature of at least 1100 °C, preferably at least 1300 °C, more preferably at least 1400 °C, in particular at least 1450 °C, to obtain the tableted alpha-alumina catalyst support.

2. The process according to claim 1, wherein the transition alumina has a loose bulk density of at most 600 g/L, a pore volume of at least 0.6 mL/g, and a median pore diameter of at least 15 nm.

3. The process according to claim 1 or 2, wherein the compacted body is dried prior to heat treating.

4. The process according to any one of the preceding claims, wherein the transition alumina comprises a phase selected from gamma-alumina, delta-alumina and theta-alumina, in particular a phase selected from gamma-alumina and delta-alumina.

5. The process according to one of the preceding claims, wherein the free-flowing feed mixture comprises, based on inorganic solids content, an alumina hydrate in an amount of at most 40 wt.-%,
wherein the alumina hydrate preferably comprises gibbsite, bayerite, boehmite and/or pseudoboehmite, preferably boehmite and/or pseudoboehmite.

6. The process according to any one of the preceding claims, wherein the pore-forming material is selected from thermally decomposable materials, burnout materials and organic polymers,
wherein the pore-forming material is preferably selected from water-soluble pore formers, in particular from oxalic acid, malonic acid, ammonium bicarbonate and/or ammonium carbonate.

7. The process according to any one of the preceding claims, wherein the free-flowing feed mixture further comprises a lubricant, wherein the lubricant is preferably selected from graphite, stearic acid and/or aluminum stearate.

8. A tableted catalyst support obtained by a process according to any one of the preceding claims.

9. The catalyst support according to claim 8, **characterized by** an alpha-alumina content of at least 85 wt.-%, a pore volume of at least 0.40 mL/g, as determined by mercury porosimetry, and a BET surface area of 0.5 to 5.0 m²/g, and wherein the pore volume contained in pores with a diameter of less than 0.1 µm preferably constitutes less than 5% of the total pore volume, as determined by mercury porosimetry.

10. The catalyst support according to claim 8 or 9, wherein at least one passageway extends from the first face side surface to the second face side surface.

11. The catalyst support according to any one of claims 8 to 10, wherein at least one of the first face side surface and the second face side surface is curved.

12. A plurality of catalyst supports according to any one of claims 8 to 11, wherein the supports have a height with no more than a 5% sample standard deviation s from the mean height, and/or wherein the supports have an outer diameter with no more than a 1% sample standard deviation s from the mean outer diameter.

13. A compacted body obtained by tableting a free-flowing feed mixture which comprises, based on inorganic solids content, at least 50 wt.-% of a transition alumina having a loose bulk density of at most 600 g/L, a pore volume of at least 0.6 mL/g, as determined by nitrogen sorption, and a median pore diameter of at least 15 nm.

14. A shaped catalyst body for producing ethylene oxide by gas-phase oxidation of ethylene, comprising at least 15 wt.-% of silver, preferably 15 to 70 wt.-% of silver, more preferably 20 to 60 wt.-% of silver, most preferably 25 to 50 wt.-% or 30 to 50 wt.-% of silver, relative to the total weight of the catalyst, deposited on a tableted alpha-alumina catalyst support according to any one of claims 8 to 12, or on a tableted alpha-alumina catalyst support obtained in the process according to any one of claims 1 to 7,
wherein the shaped catalyst body preferably comprises rhenium, in particular 400 to 2000 ppm of rhenium, relative to the total weight of the shaped catalyst body.

15. A process for producing ethylene oxide by gas-phase oxidation of ethylene, comprising reacting ethylene and oxygen in the presence of a shaped catalyst body according to claim 14.

## Patentansprüche

1. Ein Verfahren zur Herstellung eines tablettierten Alpha-Aluminiumoxid-Katalysatorträgers, umfassend:
i) das Herstellen einer frei fließenden Einsatzmischung, die - bezogen auf den anorganischen Feststoffgehalt - mindestens 50 Gew.-% einer Übergangs-Alumina umfasst, wobei die frei fließende Einsatzmischung weiterhin ein Porenbildner-Material umfasst;
ii) das Tablettieren der frei fließenden Einsatzmischung, um einen verdichteten Körper zu erhalten; und
iii) das Wärmebehandeln des verdichteten Körpers bei einer Temperatur von mindestens 1100 °C, vorzugsweise mindestens 1300 °C, stärker bevorzugt mindestens 1400 °C, insbesondere mindestens 1450 °C, um den tablettierten Alpha-Aluminiumoxid-Katalysatorträger zu erhalten.

2. Das Verfahren gemäß Anspruch 1, wobei die Übergangs-Alumina eine Schüttdichte von höchstens 600 g/L, ein Porenvolumen von mindestens 0,6 mL/g und einen mittleren Porendurchmesser von mindestens 15 nm aufweist.

3. Das Verfahren gemäß Anspruch 1 oder 2, wobei der verdichtete Körper vor der Wärmebehandlung getrocknet wird.

4. Das Verfahren gemäß einem der vorhergehenden Ansprüche, wobei die Übergangs-Alumina eine Phase umfasst, ausgewählt aus Gamma-, Delta- und Theta-Alumina, insbesondere Gamma- und Delta-Alumina.

5. Das Verfahren gemäß einem der vorhergehenden Ansprüche, wobei die frei fließende Einsatzmischung - bezogen auf den anorganischen Feststoffgehalt - ein Aluminiumhydrat in einer Menge von höchstens 40 Gew.-% umfasst, wobei das Aluminiumhydrat vorzugsweise Gibsit, Bayerit, Böhmit und/oder Pseudoböhmit umfasst.

6. Das Verfahren gemäß einem der vorhergehenden Ansprüche, wobei das Porenbildner-Material ausgewählt ist aus thermisch zersetzbaren Materialien, Ausbrennmaterialien und organischen Polymeren, bevorzugt wasserlösliche Porenbildner wie Oxalsäure, Malonsäure, Ammoniumhydrogencarbonat und/oder Ammoniumcarbonat.

7. Das Verfahren gemäß einem der vorhergehenden Ansprüche, wobei die frei fließende Einsatzmischung weiterhin ein Schmiermittel umfasst, ausgewählt aus Graphit, Stearinsäure und/oder Aluminiumstearat.

8. Ein tablettierter Katalysatorträger, erhalten durch ein Verfahren gemäß einem der vorhergehenden Ansprüche.

9. Der Katalysatorträger gemäß Anspruch 8, **gekennzeichnet durch** einen Alpha-Aluminiumoxid-Gehalt von mindestens 85 Gew.-%, ein Porenvolumen von mindestens 0,40 mL/g (Bestimmung mittels Quecksilberporosimetrie) und eine BET-Oberfläche von 0,5 bis 5,0 m²/g, wobei das Porenvolumen in Poren <0,1 µm vorzugsweise weniger als 5 % des Gesamtporenvolumens beträgt.

10. Der Katalysatorträger gemäß Anspruch 8 oder 9, wobei mindestens ein Durchgang von der ersten zur zweiten Stirnfläche reicht.

11. Der Katalysatorträger gemäß einem der Ansprüche 8-10, wobei mindestens eine der Stirnflächen gekrümmt ist.

12. Eine Vielzahl von Katalysatorträgern gemäß einem der Ansprüche 8-11, wobei die Träger eine Höhe mit höchstens 5 % Stichproben-Standardabweichung s vom Mittelwert und/oder einen Außendurchmesser mit höchstens 1 % Stichproben-Standardabweichung s aufweisen.

13. Ein verdichteter Körper, erhalten durch das Tablettieren einer frei fließenden Einsatzmischung, die - bezogen auf den anorganischen Feststoffgehalt - mindestens 50 Gew.-% einer Übergangs-Alumina mit Schüttdichte ≤600 g/L, Porenvolumen ≥0,6 mL/g (Stickstoffsorption) und mittlerem Porendurchmesser ≥15 nm umfasst.

14. Ein geformter Katalysatorkörper zur Herstellung von Ethylenoxid durch Gasphasenoxidation von Ethylen, umfassend mindestens 15 Gew.-% Silber, vorzugsweise 15-70 Gew.-%, stärker bevorzugt 20-60 Gew.-%, besonders bevorzugt 25-50 Gew.-% oder 30-50 Gew.-%, abgeschieden auf einem tablettierten Alpha-Aluminiumoxid-Katalysatorträger gemäß einem der Ansprüche 8 bis 12 oder auf einem tablettierten Alpha-Aluminiumoxid-Katalysatorträger, der in dem Verfahren gemäß einem der Ansprüche 1 bis 7 hergestellt wurde,
wobei der geformte Katalysatorkörper vorzugsweise Rhenium umfasst, insbesondere 400 bis 2000 ppm Rhenium, bezogen auf das Gesamtgewicht des geformten Katalysatorkörpers.

15. Ein Verfahren zur Herstellung von Ethylenoxid durch Gasphasenoxidation von Ethylen unter Einsatz eines geformten Katalysatorkörpers gemäß Anspruch 14.

## Revendications

1. Un procédé de production d'un support de catalyseur en alpha-alumine comprimée, comprenant :
i) la formation d'un mélange d'alimentation à écoulement libre comprenant, sur la base de la teneur en solides inorganiques, au moins 50 % en poids d'une alumine de transition, ledit mélange comprenant en outre un agent formateur de pores ;
ii) la compression du mélange d'alimentation à écoulement libre pour obtenir un corps compacté ; et
iii) le traitement thermique du corps compacté à une température d'au moins 1100 °C, de préférence au moins 1300 °C, plus préférentiellement au moins 1400 °C, en particulier au moins 1450 °C, afin d'obtenir le support de catalyseur en alpha-alumine comprimée.

2. Le procédé selon la revendication 1, dans lequel l'alumine de transition présente une densité apparente maximale de 600 g/L, un volume poreux d'au moins 0,6 mL/g et un diamètre moyen de pores d'au moins 15 nm.

3. Le procédé selon l'une quelconque des revendications 1 ou 2, dans lequel le corps compacté est séché avant le traitement thermique.

4. Le procédé selon l'une quelconque des revendications précédentes, dans lequel l'alumine de transition comprend une phase choisie parmi la gamma-alumine, la delta-alumine et la thêta-alumine, en particulier parmi la gamma-alumine et la delta-alumine.

5. Le procédé selon l'une quelconque des revendications précédentes, dans lequel le mélange d'alimentation à écoulement libre comprend, sur la base de la teneur en solides inorganiques, un hydrate d'alumine en une quantité d'au plus 40 % en poids, l'hydrate d'alumine comprenant de préférence la gibbsite, la bayerite, la boehmite et/ou la pseudoboehmite.

6. Le procédé selon l'une quelconque des revendications précédentes, dans lequel l'agent formateur de pores est choisi parmi des matériaux thermiquement décomposables, des matériaux combustibles (burnout) et des polymères organiques, de préférence parmi les agents formateurs de pores hydrosolubles choisis en particulier parmi l'acide oxalique, l'acide malonique, le bicarbonate d'ammonium et/ou le carbonate d'ammonium.

7. Le procédé selon l'une quelconque des revendications précédentes, dans lequel le mélange d'alimentation à écoulement libre comprend en outre un lubrifiant, choisi de préférence parmi le graphite, l'acide stéarique et/ou le stéarate d'aluminium.

8. Un support de catalyseur comprimé obtenu par un procédé selon l'une quelconque des revendications précédentes.

9. Le support de catalyseur selon la revendication 8, **caractérisé par** une teneur en alpha-alumine d'au moins 85 % en poids, un volume poreux d'au moins 0,40 mL/g déterminé par porosimétrie au mercure, et une surface BET comprise entre 0,5 et 5,0 m²/g, le volume poreux contenu dans les pores d'un diamètre inférieur à 0,1 µm représentant de préférence moins de 5 % du volume poreux total.

10. Le support de catalyseur selon l'une quelconque des revendications 8 ou 9, dans lequel au moins un passage traverse la surface latérale d'un premier côté à celle d'un second côté.

11. Le support de catalyseur selon l'une quelconque des revendications 8 à 10, dans lequel au moins l'une des surfaces latérales est incurvée.

12. Une pluralité de supports de catalyseur selon l'une quelconque des revendications 8 à 11, lesdits supports présentant une hauteur avec un écart-type d'échantillon ne dépassant pas 5 % de la hauteur moyenne et/ou un diamètre extérieur avec un écart-type d'échantillon ne dépassant pas 1 % du diamètre extérieur moyen.

13. Un corps compacté obtenu par compression d'un mélange d'alimentation à écoulement libre comprenant, sur la base de la teneur en solides inorganiques, au moins 50 % en poids d'une alumine de transition ayant une densité apparente maximale de 600 g/L, un volume poreux d'au moins 0,6 mL/g (déterminé par adsorption d'azote) et un diamètre moyen de pores d'au moins 15 nm.

14. Un corps de catalyseur façonné pour la production d'oxyde d'éthylène par oxydation en phase gazeuse de l'éthylène, comprenant au moins 15 % en poids d'argent, de préférence 15 à 70 % en poids d'argent, plus préférentiellement 20 à 60 % en poids d'argent, très préférentiellement 25 à 50 % ou 30 à 50 % en poids d'argent, par rapport au poids total du catalyseur, déposé sur un support de catalyseur en alpha-alumine comprimée selon l'une des revendications 8 à 12 ou obtenu selon l'une des revendications 1 à 7, ledit corps de catalyseur comprenant de préférence du rhénium, en particulier 400 à 2000 ppm de rhénium, par rapport au poids total du corps de catalyseur façonné.

15. Un procédé de production d'oxyde d'éthylène par oxydation en phase gazeuse de l'éthylène, comprenant la réaction de l'éthylène et de l'oxygène en présence d'un corps de catalyseur façonné selon la revendication 14.
